# EUROPÄISCHE PATENTANMELDUNG

(11) **EP 2 060 625 A1**
(43) Veröffentlichungstag der Anmeldung: **20.05.2009**
(21) Anmeldenummer: 07120917.5
(22) Anmeldetag: 16.11.2007
(51) Int. Cl.: C12M 1/26

(54) **Vorrichtung zur Elution von zur Analyse vorgesehenen Proben**

(71) Anmelder: Dentognostics GMBH, 07745 Jena (DE)
(72) Erfinder: Sältzer, Jan, 07745 Jena (DE); Pietraszcyk, Marko, 07747 Jena (DE)
(74) Vertreter: Meyers, Hans-Wilhelm

(57) **Zusammenfassung**

Gegenstand der Erfindung ist eine Vorrichtung zur Elution von zur Analyse vorgesehenen Proben, die auf einem Probenträger (20) absorbiert sind. Diese umfasst einen Behälter (10) mit einer Wandung 30), einer inneren und äußeren Oberfläche, und mit wenigstens einer Öffnung, die als Einlass- (40) und/oder Auslassöffnung (50) ausgebildet ist, und eine Öffnung zur Aufnahme eines verschiebbaren Kolbenelementes (60) mit einem Stempel (70) und einer Kolbenstange (80), so dass das Kolbenelement (60) den Behälter verschließt und die Einlass- (40) und/oder Auslassöffnung (50) in einem dem Kolbenelement 60) gegenüber angeordneten Bereich (90) vorgesehen ist.

## Beschreibung

Die Erfindung betrifft eine Vorrichtung zur Elution von Proben, ein Verfahren zur Probenelution und die Verwendung der Vorrichtung, sowie eine Elutionsflüssigkeit, welche in diesem Verfahren eingesetzt wird.

Im Allgemeinen verfolgt man mit einer Extraktion zwei Hauptziele. Eines ist die Extraktion eines Analyten mit hoher Ausbeute und Reinheit, und das andere ist eine hohe Extraktions- und Elutionsgeschwindigkeit.
Ein spezieller Typ der Extraktion ist die Festphasenextraktion (Solid phase extraction, SPE). SPE ist ein Verfahren der Probenvorbereitung, bei dem ein Analyt durch Absorption oder Adsorption an einem Festphasenmedium aus einer flüssigen Probe entfernt und konzentriert wird. Dann erfolgt die Elution des Analyten mit einem für die Analyse geeignetem Lösungsmittel.
Ein weitere Anreicherungstechnik ist die Festphasenmikroextraktion (Solid phase micro extraction, SPME), die auf einem ähnlichen Prinzip basiert wie die SPE. Sie eignet sich jedoch überwiegend zur Extraktion leichtflüchtiger Verbindungen. Ähnlich wie bei der SPE findet auch hier zunächst die Adsorption eines Analyten an eine feste Phase geeigneter Polarität statt. Im Vergleich zur Festphasenextraktion wird der Analyt bei der SPME jedoch nicht vollständig aus der flüssigen Probe extrahiert. Die Elution des Probenmaterials erfolgt vielmehr im sog. steady-state-Verfahren. Bei diesem Verfahren bildet sich nach einer definierten Zeit ein Fließgleichgewicht von absorbiertem und in Lösung befindlichem Analyt.

Entscheidend beim SPME-Verfahren ist, dass die Extraktion nicht vollständig verläuft, sondern es sich lediglich um einen Gleichgewichtsprozess handelt, bei dem ein bestimmter Anteil eines Analyten - entsprechend dem Verteilungskoeffizienten des Analyten zwischen fester Phase und Eluent - an der Festphase absorbiert wird. Dabei muss die Einstellung des Fließgleichgewichts nicht abgewartet werden, solange die Extraktionszeit konstant gehalten wird, so dass eine gleichmäßige Diskriminierung des Analyten in den zu messenden Proben erfolgt.

Verwendung finden die oben genannten Festphasenextraktionsmethoden u. a. in der zahnmedizinischen Analytik, insbesondere beim Monitoring von Gingivitis- und Parodontitis-Erkrankungen. Gingivitis und Parodontitis werden durch den Dauerreiz des dentalen Biofilms ausgelöst. Ob allerdings eine Parodontitis tatsächlich eintritt, wird durch die Gegenreaktion des Wirtsorganismus bestimmt. Für den Beginn und das Fortschreiten einer Parodontitis wie auch des alveolären Knochenabbaus sind hierbei wirtseigene Kollagenasen verantwortlich.

Die für parodontopathogene Abbauprozesse wichtigste Kollagenase ist die Matrix-Metalloproteinase-8 (MMP-8), synonym für Kollagenase 2.

Matrixmetalloproteinasen liegen in drei verschiedenen Formen vor:
1. Inaktive Vorform (Speicherform in polymorphkernigen Granulozyten)
2. Aktivierte bzw. aktive Form
3. Inhibierte Form.

Die im Gewebe freigesetzte aktive Form (aMMP-8) ist letztendlich verantwortlich für die Gewebedestruktion. Hohe aMMP-8-Spiegel weisen auf eine aktive Entzündung und damit auf einen akuten, behandlungsbedürftigen Zustand hin.

aMMP-8 ist ein objektiver, diagnostischer Marker zur Erkennung des Zeitpunkts, zu dem parodontales Gewebe abgebaut wird.

So wird z. B. Sulkusfluid aus den Gingivaltaschen im Mundraum des Patienten mittels eines Probenträgers aufgesaugt. Dazu werden dünne Membranen aus PVDF (Polyvinylidenfluorid) verwendet. Hierzu wird der Probenträger für ca. 30 Sekunden in eine Gingivaltasche des Patienten eingebracht.
Anschließend wird eine Elution des adsorbierten Analyten durchgeführt. Dazu wird der Probenträger in ein flüssiges Elutionsmedium überführt. Die Elution des Analyten/Probenmaterials erfolgt im steady-state-Verfahren. Das eluierte Probenmaterial wird darauf mittels eines immunologischen Verfahrens (Munjal et al., Ann N Y Acad Sci, 2007, 1098, 486-89; Prescher et al., Ann N Y Acad Sci, 2007, 1098, 493-95; Munjal et al., Ann N Y Acad Sci, 2007, 1098, 490-92) auf das Vorhandensein von Krankheitsmarkern untersucht, die auf eine Parodontitis-Erkrankung hinweisen. Die Probennahme und die Extraktion wird dabei von fachkundigem Personal manuell durchgeführt.

Die Festphasenextraktionsmethoden des Stands der Technik weisen folgende Nachteile auf:
Eine unvollständige Einstellung des Fließgleichgewichts kann zu einer undefinierten Probenelution führen, was die Wiederholbarkeit und Robustheit der Nachweismethode beeinträchtigen kann.
Ferner können schon geringe Abweichungen der Extraktionszeit zu fehlerbehafteten Messwerten führen. Es ist daher wünschenswert, über eine Methode zu verfügen, die auch von ungeschultem Personal angewendet werden kann, um verlässliche Messwerte zu erhalten.
Außerdem ist es bisher nicht möglich, die Probenstreifen nach Probennahme über einen längeren Zeitraum zu lagern. Bei Lagerung besteht die Gefahr, dass eine Verminderung von defektionsfähigen Analyten und damit eine Verfälschung der Analysenergebnisse nicht auszuschließen ist.

Überraschenderweise werden diese Nachteile mit der erfindungsgemäßen Vorrichtung und dem erfindungsgemäßen Verfahren überwunden. Gegenstand der Erfindung ist dabei eine Vorrichtung zur Elution von zur Analyse vorgesehenen Proben, die auf einem Probenträger absorbiert sind. Diese umfasst einen Behälter mit einer Wandung, einer inneren und äußeren Oberfläche, und mit wenigstens einer Öffnung, die als Einlass- und/oder Auslassöffnung ausgebildet ist, und eine Öffnung zur Aufnahme eines verschiebbaren Kolbenelementes mit einem Stempel und einer Kolbenstange, so dass das Kolbenelement den Behälter verschließt und die Einlass- und/oder Auslassöffnung in einem dem Kolbenelement gegenüber angeordneten Bereich vorgesehen ist.
Die Vorrichtung ist dadurch gekennzeichnet, dass im Volumen des Behälters mindestens zwei Sperrelemente vorhanden sind, wobei sich ein erstes Sperrelement hinter dem Stempel und vom Stempel lösbar angeordnet und sich ein zweites Sperrelement vor der Einlass- oder Auslassöffnung befindet und in der Wandung zwischen dem ersten und dem zweiten Sperrelement eine Aufnahmeeinrichtung für den Probenträger vorgesehen ist.

### Kurzbeschreibung der Figuren

Fig. 1 ist ein Querschnitt der erfindungsgemäßen Vorrichtung.
Fig. 2a zeigt wie der Probenträger 20 durch Aufnahmeeinrichtung 120 in die Vorrichtung eingegeben wird.
Fig. 2b illustriert die Fixierung des Probenträgers 20 zwischen den Sperrelementen 100 und 110.
Fig. 2c zeigt, wie das Kolbenelement 60 im Behälter 10 zurückgezogen wird.
Fig. 2d zeigt, wie Elutionsflüssigkeit durch die Einlassöffnung 40 zugegeben wird und der Probenträger 20 umspült wird, wobei über die Aufnahmevorrichtung 120 Luft entweichen kann.
Fig. 2e illustriert die Gewinnung des Eluats durch die Auslassöffnung 50.

In einer besonderen Ausführungsform der erfindungsgemäßen Vorrichtung ist die Aufnahmeeinrichtung 120 näher an dem Kolbenelement 60, als an dem Kolbenelement gegenüber befindlichem Bereich 90 angeordnet.

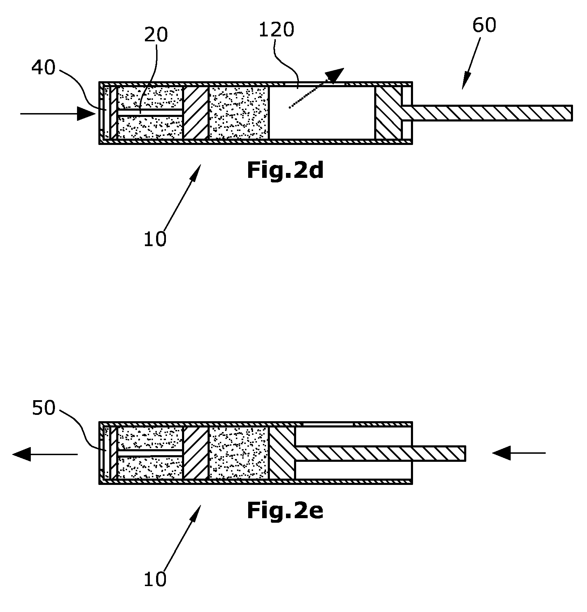

In einer weiteren Ausführungsform der erfindungsgemäßen Vorrichtung ist das erste Sperrelement 100 bei Zugabe der Elutionsflüssigkeit ohne Kontakt zum Stempel 70 nicht verschiebbar (vgl. Figur 2d). Dies kann dadurch gewährleistet sein, dass das Sperrelement 100 so in den Behälter eingepasst ist, dass es ausreichend schwergängig verschiebbar ist, um der einströmenden Flüssigkeit zu widerstehen.

In einer zusätzlichen Ausführungsform sind die Sperrelemente 100 und 110 chemisch inert, d. h. es erfolgt keine chemische Reaktion mit der Elutionsflüssigkeit oder dem Probenträger 20. Weiterhin können die Sperrelemente Filter sein.

Der Behälter 10 und die Sperrelemente 100 und 110 der erfindungsgemäßen Vorrichtung sind bevorzugt aus Kunststoff oder Glas gefertigt, wobei diese möglichst keine chemische Reaktion mit dem Probenträger 20 oder der Elutionsflüssigkeit eingehen. Eine geringe Diffusion von Weichmachern des Kunststoffes in die Elutionslösung ist dabei technisch nicht ausschließbar.

Als Kunststoff können dabei beispielsweise Polyethylen, Polypropylen, Polyvinylchlorid, Polymethylmethacrylat, Polyacrylat oder Polyethylenterephthalat oder Mischungen von Polymeren sowie Verbundstoffe (Kunststoffe mit Metallbeschichtungen etc.) verwendet werden. Diese können Weichmacher, Additive und weitere Hilfsstoffe enthalten.
In einer weiteren Ausführungsform ist die erfindungsgemäße Vorrichtung wiederverwendbar, indem der Behälter 10 aus sterilisierbarem Glas besteht. Weiterhin kann es sich bei den Sperrelementen 100 und 110 um Glasfritten handeln, welche ebenfalls sterilisierbar sind. Das Kolbenelement kann wiederum aus sterilisierbarem Glas bestehen.

In einer zusätzlichen Ausführungsform besteht der erfindungsgemäße Probenträger 20 aus einem Material mit großer Oberfläche, an die eine Absorption von organischen Molekülen wie Peptiden oder Proteinen möglich ist. Der Probenträger 20 besteht dabei beispielsweise aus Papier, aus Kunststoff oder aus einer Metallfolie, wobei eine Beschichtung aufgetragen sein kann. Bei dem Kunststoff handelt es sich beispielsweise um Polyvinylidenfluorid. Bei der Beschichtung kann es sich zum Beispiel um eine Kunststoffbeschichtung oder eine Beschichtung mit Kieselgel, Aluminiumoxid oder einer Umkehrphase (RP-Phase) handeln.
Darüber hinaus ist ein Verfahren zur Elution von Proben, insbesondere von Proteinen, von mindestens einem Probenträger 20 Gegenstand der Erfindung, wobei
(a) der Probenträger 20 über die Aufnahmeeinrichtung 120 in der Wandung in die Vorrichtung gemäß Anspruch 1 eingebracht,
(b) der Probenträger mittels Kolbenelement 60 zwischen den Sperrelementen 100 und 110 in der Vorrichtung fixiert,
(c) das Kolbenelement 60 zurückgezogen,
(d) der Probenträger 20 von einer Elutionsflüssigkeit, welche durch die Einlassöffnung 40 zugegeben wird, umströmt,
(e) die Elutionsflüssigkeit mit dem Kolbenelement 60 durch die Auslassöffnung 50 nahezu vollständig aus der Vorrichtung verdrängt, und
(f) in ein Gefäß überführt und mit analytischen Methoden untersucht wird.

Dabei wird der Probenträger 20 im Durchfluss eluiert, was zu einer schnelleren und effizienteren Extraktion im Vergleich zum o. g. steady-state-Verfahren führt.

In einer weiteren Ausführungsform stellt die vorliegende Erfindung ein Extraktionsverfahren bereit, wobei die verdrängte Luft durch die Aufnahmeinrichtung 120 austreten kann, so dass die Elutionsflüssigkeit praktisch frei von Luftblasen ist. In einer zusätzlichen Ausführungsform ist die Aufnahmeeinrichtung 120 so ausgestaltet, dass zwar die verdrängte Luft, nicht jedoch die Elutionsflüssigkeit durch die Aufnahmeeinrichtung 120 austreten kann. Dies kann z. B. durch einen geeigneten gasdurchlässigen Verschluss erfolgen.

Als analytische Methoden kommen dabei alle gängigen Verfahren der Chromatographie (u. a. HPLC (High Performance Liquid Chromatographie), GC (Gaschromatographie), DC (Dünnschichtchromatographie)), der immunologischen Analyse, der Elektrophorese (u. a. Kapillarelektrophorese, Gelelektrophorese) und der Spektrometrie (u. a. MS, IR, UV) in Frage.

So kann eine quantitative immunologische Analyse der gewonnen Proteine z. B. mittels ELISA (Enzyme-linked immunosorbent assay) oder IFMA (Immunofluorometric assay) durchgeführt werden. Ferner ist eine quantitative immunologische Analyse auch nach oben beschriebenem Verfahren möglich. Eine qualitative immunologische Analyse ist mittels Westernblot möglich.
Bei der Elutionsflüssigkeit des erfindungsgemäßen Verfahrens kann es sich um eine polare Flüssigkeit handeln, die zur Elution von Peptiden oder Proteinen geeignet ist, wie z. B. wässrige Pufferlösungen mit saurem, neutralem oder basischem pH-Wert, einer alkoholischen Lösung, einem Ether, Ester, Keton oder Nitril. Beispielsweise kann Methanol, Ethanol, n-Propanol, i-Propanol, Ethylacetat, Acetonitril, Wasser, Ammoniak, Ameisensäure, Essigsäure, Diethylether, Aceton, Dichlormethan, Trichlormethan, Dimethylsulfoxid, Dimethylformamid, Pyridin oder eine Mischung oder Lösung davon eingesetzt werden.

Ferner können mit dem erfindungsgemäßen Verfahren auch apolare oder eher apolare Stoffe von einem Probenträger eluiert werden. Dafür können apolare Lösungsmittel als Elutionslösung wie beispielsweise n-Pentan, n-Hexan, Toluol, Benzol oder Diethylether und Mischungen davon verwendet werden.

Die erfindungsgemäße Vorrichtung gemäß Anspruch 1 kann zur Elution von Proteinen, insbesondere Matrix-Metalloproteasen (MMP), von mindestens einem Probenträger verwendet werden.

Ferner kann die erfindungsgemäße Vorrichtung gemäß Anspruch 1 in der medizinischen Analytik, insbesondere in der zahnmedizinischen Analytik verwendet werden. Darüber hinaus ist eine Verwendung in der Lebensmittelanalytik, der sportmedizinischen Analytik und in der Umweltanalytik möglich.

Außerdem eignet sich die erfindungsgemäße Vorrichtung zur Aufbewahrung mindestens eines Probenträgers.

Gegenstand der Erfindung ist auch eine Elutionsflüssigkeit folgender Zusammensetzung:
- 10 bis 50 mM, oder 20 bis 40 mM, oder 25 bis 35 mM Na₂HPO₄* 12 H₂O;
- 2 bis 20 mM, oder 5 bis 15 mM, oder 7 bis 13 mM NaH₂PO₄* H₂O;
- 50 bis 900, oder 100 bis 700 mM, oder 250 bis 500 mM mM NaCl;
- 0,05 Vol.-% 5-Brom-5-nitro-1,3-dioxan;
- 0,01 bis 0,5 Vol.-%, oder 0,1 bis 0,2 Vol.-% Rinderserumalbumin (BSA);
- 0,005 bis 0,5 Vol.-%, oder 0,05 bis 0,2 Vol.-% Tween20 (Polysorbat 20)
oder Tetronic 1307 (BASF AG, Blockcopolymer aus Ethylenoxid/Propylenoxid). Die erfindungsgemäße Elutionsflüssigkeit kann im erfindungsgemäßen Verfahren eingesetzt werden.
Ferner kann die erfindungsgemäße Elutionsflüssigkeit zur Elution von Proteinen, insbesondere Matrix-Metalloproteasen (MMP), von mindestens einem Probenträger (20) eingesetzt werden.

### Ausführliche Beschreibung der Figuren

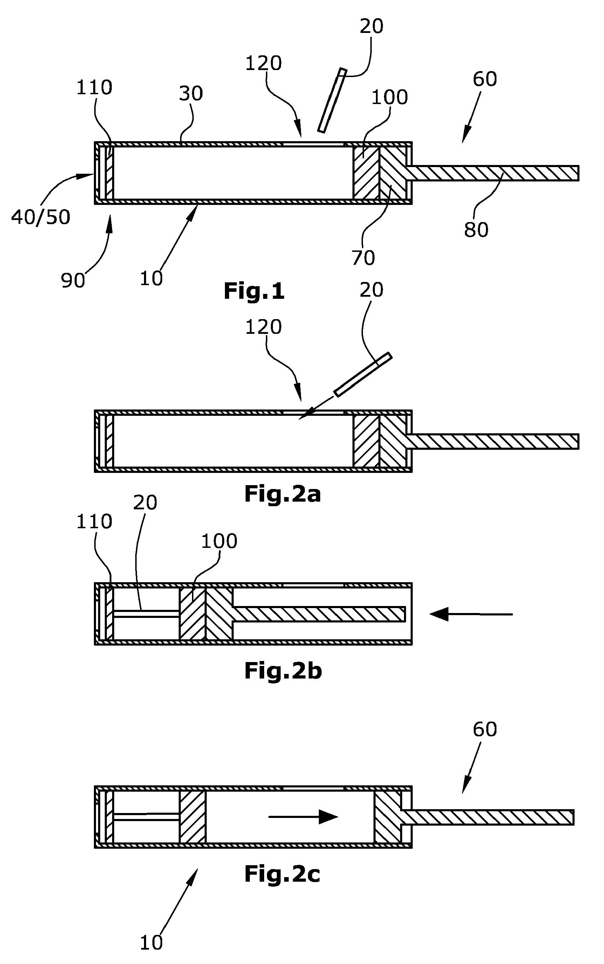

Figur 1 zeigt die Vorrichtung zur Elution von zur Analyse vorgesehenen Proben, die auf einem Probenträger 20 absorbiert sind. Die Vorrichtung umfasst einen Behälter 10 mit einer Wandung 30, einer inneren und äußeren Oberfläche, und mit wenigstens einer Öffnung, die als Einlass- 40 und/oder Auslassöffnung 50 ausgebildet ist. Eine weitere Öffnung dient zur Aufnahme eines verschiebbaren Kolbenelementes 60 mit einem Stempel 70 und einer Kolbenstange 80. Dabei verschließt das Kolbenelement 60 den Behälter. Ferner ist in einem dem Kolbenelement 60 gegenüber angeordneten Bereich 90 die Einlass- 40 und/oder Auslassöffnung 50 vorgesehen.
Die Vorrichtung ist dadurch gekennzeichnet, dass im Volumen des Behälters mindestens zwei Sperrelemente 100 und 110 vorhanden sind, wobei sich ein erstes Sperrelement 100 hinter dem Stempel 70 und vom Stempel 70 lösbar angeordnet und sich ein zweites Sperrelement 110 vor der Einlass- 40 und/oder Auslassöffnung 50 befindet. Außerdem weist die Wandung 30 zwischen dem ersten und dem zweiten Sperrelement 100 und 110 eine Aufnahmeeinrichtung 120 für den Probenträger 20 auf.

In Figur 2a ist dargestellt, wie der Probenträger 20 in den Behälter 10 durch die Aufnahmeeinrichtung 120 gegeben wird. Dabei handelt es sich bei der Aufnahmeeinrichtung 120 um eine Öffnung in der Wandung 30 des Behälters 10. Das Kolbenelement 60 und das Sperrelement 100 befinden sich dabei möglichst weit gegenüber des Bereichs 90, so dass der Probenträger 20 in den Behälter 10 gegeben werden kann.

Figur 2b beschreibt, wie der Probenträger 20 durch eine axiale Bewegung (in Pfeilrichtung) des Kolbenelements 60 und des Sperrelemts 100 in Richtung des Endes, welches die Einlassöffnung 40 aufweist, zwischen den beiden Sperrelementen 100 und 110 fixiert wird. Dabei ist darauf zu achten, dass der Probenträger 20 nicht an die Wandung 30 anhaftet, was eine vollständige Elution erschweren würde.

Fig. 2c zeigt wie das Kolbenelement 60 im Behälter 10 zurückgezogen wird. Dabei bleibt der Probenträger zwischen den beiden Sperrelementen 100 und 110 fixiert und das Sperrelement 100 löst sich vom Kolbenelement 60.

Daran anschließend wird, wie in Figur 2d dargestellt, Elutionsflüssigkeit durch die Einlassöffnung 40 zugegeben, welche den Probenträger im Rückfluss 20 umspült. Dabei wird das Sperrelement 100 nicht durch die einströmende Flüssigkeit verschoben, wodurch der Probenträger 20 fixiert bleibt. Eine umfassende Elution im Rückfluss wird dadurch gewährleistet. Die Zugabe von Elutionsflüssigkeit erfolgt beispielsweise mit einer Spritze, einer Pipette oder über eine Schlauchverbindung.
Fig. 2e illustriert wie hierauf das Eluat mit dem Kolbenelement 60 durch die Auslassöffnung 50 in ein Gefäß überführt wird. Dabei wird die Elutionsflüssigkeit nahezu vollständig aus dem Behälter 10 verdrängt.

Das erfindungsgemäße Verfahren wird im Folgenden beispielhaft dargestellt:
Der Probenträger 20, auch Probenahmestreifen genannt, wird für 30 Sek. in eine Gingivaltasche (Zwischenraum von Zahn und Zahnfleisch) eines Patienten eingeführt, wobei Sulkusfluid in den Probenträger diffundiert. Dieser wird mit einer Pinzette entnommen, über die Aufnahmeeinrichtung 120 in den Behälter 10 eingebracht (vgl. Figur 2a) und mit dem Kolbenelement 60 zwischen den Sperrelementen 100 und 110 im dem Kolbenelement 60 gegenüber angeordneten Bereich 90 fixiert (vgl. Fig. 2b).
Ferner werden 800 µl einer osmotischen Elutionsflüssigkeit mit einer Pipette durch die Einlassöffnung 40 zugegeben, welche das Sulkusfluid zusammen mit dem Entzündungsmarker MMP-8 vom Probenträger 20 eluiert.

Zusammensetzung der wässrigen Elutionsflüssigkeit (alle Prozentangaben in Volumenprozent):
- 15 mM Na₂HPO₄* 12 H₂O;
- 7 mM NaH₂PO₄* H₂O;
- 550 mM NaCl;
- 0,05% 5-Brom-5-nitro-1,3-dioxan;
- 0,2% Rinderserumalbumin (BSA);
- 0,3% Tween 20 (Polysorbat 20) oder Tetronic 1307 (BASF AG, Blockcopolymer aus Ethylenoxid/Propylenoxid).

Dabei wird der Probenträger 20 im Durchfluss eluiert. Anschließend wird das Eluat über die Auslassöffnung 50 mit dem Kolbenelement 60 in ein Probenröhrchen überführt.
Die so erhaltene Probelösung wird in einem MMP-8 sensitiven ELISA quantitativ analysiert. Eine Untersuchung des Eluats nach den Verfahren von Prescher et al. und Munjal et al. (Prescher et al., Ann N Y Acad Sci, 2007, 1098, 493-95; Munjal et al., Ann N Y Acad Sci, 2007, 1098, 490-92) wird ebenfalls durchgeführt.

## Patentansprüche

1. Vorrichtung zur Elution von zur Analyse vorgesehenen Proben, die auf einem Probenträger (20) absorbiert sind, umfassend einen Behälter (10) mit einer Wandung (30), einer inneren und äußeren Oberfläche, und mit wenigstens einer Öffnung, die als Einlass- (40) und/oder Auslassöffnung (50) ausgebildet ist, und eine Öffnung zur Aufnahme eines verschiebbaren Kolbenelementes (60) mit einem Stempel (70) und einer Kolbenstange (80), so dass das Kolbenelement (60) den Behälter verschließt und die Einlass- (40) und/oder Auslassöffnung (50) in einem dem Kolbenelement (60) gegenüber angeordneten Bereich (90) vorgesehen ist,
**dadurch gekennzeichnet, dass**
im Volumen des Behälters mindestens zwei Sperrelemente (100, 110) vorhanden sind, wobei sich ein erstes Sperrelement (100) hinter dem Stempel (70) und vom Stempel (70) lösbar angeordnet und sich ein zweites Sperrelement (110) vor der Einlass- (40) oder Auslassöffnung (50) befindet und in der Wandung (30) zwischen dem ersten und dem zweiten Sperrelement (100, 110) eine Aufnahmeeinrichtung (120) für den Probenträger (20) vorgesehen ist.

2. Vorrichtung gemäß Anspruch 1, **dadurch gekennzeichnet, dass** die Aufnahmeeinrichtung (120) näher an dem Kolbenelement (60) als an dem Kolbenelement gegenüber befindlichen Bereich (90) angeordnet ist.

3. Vorrichtung gemäß Anspruch 1 und/oder 2, **dadurch gekennzeichnet, dass** das Sperrelement (100) bei Zugabe der Elutionsflüssigkeit ohne Kontakt zum Stempel (70) nicht verschiebbar ist.

4. Vorrichtung gemäß mindestens einem der Ansprüche 1-3, **dadurch gekennzeichnet, dass** die Sperrelemente (100, 110) chemisch inert sind.

5. Vorrichtung gemäß mindestens einem der Ansprüche 1-4, **dadurch gekennzeichnet, dass** die Sperrelemente (100, 110) Filter sind.

6. Verfahren zur Elution von Proben, insbesondere von Proteinen, von mindestens einem Probenträger (20), **dadurch gekennzeichnet, dass**
a. der Probenträger (20) über die Aufnahmeeinrichtung (120) in der Wandung (30) in die Vorrichtung gemäß Anspruch 1 eingebracht,
b. der Probenträger (20) mittels Kolbenelement (60) zwischen den Sperrelementen (100, 110) in der Vorrichtung fixiert,
c. das Kolbenelement (60) zurückgezogen,
d. eine Elutionsflüssigkeit, welche durch die Einlassöffnung (40) zugegeben wird, den Probenträger (20) umströmt,
e. die Elutionsflüssigkeit mit dem Kolbenelement (60) durch die Auslassöffnung (50) nahezu vollständig aus der Vorrichtung verdrängt, und
f. in ein Gefäß überführt und mit analytischen Methoden untersucht wird.

7. Verfahren gemäß Anspruch 6, **dadurch gekennzeichnet, dass**
die verdrängte Luft durch die Aufnahemeinrichtung (120) austreten kann, so dass die Elutionsflüssigkeit praktisch frei von Luftblasen ist.

8. Verfahren gemäß Anspruch 6 und/oder 7, **dadurch gekennzeichnet, dass** die Elutionsflüssigkeit folgende Komponenten umfasst:
- 10 bis 50 mM Na₂HPO₄* 12 H₂O;
- 2 bis 20 mM NaH₂PO₄* H₂O;
- 50 bis 900 mM NaCl;
- 0,05 Vol.-% 5-Brom-5-nitro-1,3-dioxan;
- 0,01 bis 0,5 Vol.-% Rinderserumalbumin (BSA);
- 0,005 bis 0,5 Vol.-% Tween20 (Polysorbat 20) oder Tetronic 1307 (BASF AG, Blockcopolymer aus Ethylenoxid/Propylenoxid).

9. Verwendung der Vorrichtung gemäß Anspruch 1 zur Elution von Proteinen, insbesondere Matrix-Metalloproteasen (MMP), von mindestens einem Probenträger (20).

10. Verwendung der Vorrichtung gemäß Anspruch 1 zur Aufbewahrung mindestens eines Probenträgers (20).

11. Elutionsflüssigkeit enthaltend:
- 10 bis 50 mM Na₂HPO₄* 12 H₂O;
- 2 bis 20 mM NaH₂PO₄* H₂O;
- 50 bis 900 mM NaCl;
- 0,05 Vol.-% 5-Brom-5-nitro-1,3-dioxan;
- 0,01 bis 0,5 Vol.-% Rinderserumalbumin (BSA);
- 0,005 bis 0,5 Vol.-% Tween20 (Polysorbat 20) oder Tetronic 1307 (BASF AG, Blockcopolymer aus Ethylenoxid/Propylenoxid).

12. Verwendung der Elutionsflüssigkeit gemäß Anspruch 11 zur Elution von Proteinen, insbesondere Matrix-Metalloproteasen (MMP), von mindestens einem Probenträger (20).
